# EUROPEAN PATENT APPLICATION

(11) **EP 4 840 876 A1**
(43) Date of publication of application: **18.11.2026**
(21) Application number: 25914216.4
(22) Date of filing: 21.02.2025
(51) Int. Cl.: C07K 14/245, C12N 15/31, C12N 1/21, C12P 13/08

(54) **MUTANT YBII PROTEIN AND GENE THEREOF, RECOMBINANT STRAIN, USE, AND PRODUCTION METHOD**

(30) Priority: 03.01.2025 CN 202510007464
(71) Applicant: Hulunbeier Northeast Fufeng Biotechnologies Co., Ltd, Hulun Buir, Inner Mongolia 162650 (CN); QIQIHAR LONGJIANG FUFENG BIOTECHNOLOGIES CO., LTD., Qiqihar, Heilongjiang 161031 (CN)
(72) Inventor: WANG, Juncheng, Inner Mongolia 162650 (CN); XU, Zhiying, Inner Mongolia 162650 (CN); FANG, Ning, Inner Mongolia 162650 (CN); LI, Siqi, Inner Mongolia 162650 (CN); CHENG, Xi, Inner Mongolia 162650 (CN); LIU, Yuantao, Inner Mongolia 162650 (CN); LIU, Shizhou, Inner Mongolia 162650 (CN); SHI, Shuzhi, Inner Mongolia 162650 (CN); LIU, Chengcai, Qiqihar, Heilongjiang 161031 (CN); ZHAO, Lankun, Qiqihar, Heilongjiang 161031 (CN)
(74) Representative: Tiburzi, Andrea
(86) International application number: PCT/CN2025/078544
(87) International publication number: WO 2026/143820

(57) **Abstract**

The present invention provides a mutant YbiI protein and a gene thereof, a recombinant strain, a use, and a production method. Relative to a wild-type YbiI protein represented by SEQ ID NO: 1, the mutant YbiI protein at least comprises the following mutation site: an amino acid at position 86 is mutated from glutamine to leucine, and the mutation can increase the yield of threonine in different strains.

## Description

### TECHNICAL FIELD

The present application belongs to the field of microbial fermentation and specifically relates to a mutant YbiI protein and a gene thereof, a recombinant strain, use, and a production method.

### BACKGROUND

As one of the eight amino acids for the growth of human beings and animals, threonine has been widely used in medicine, chemical reagents, food fortifiers, feed additives, and other fields. Threonine is an important nutritional enhancer that can be used in cereals, pastries, and dairy products. Like tryptophan, it has the effect of relieving fatigue and promoting growth and development. In medicine, due to the presence of a hydroxyl group in the structure of threonine, threonine has a water-retaining effect on human skin and, when combined with oligosaccharide chains, plays an important role in protecting cell membranes and promotes phospholipid synthesis and fatty acid oxidation *in vivo*. Currently, threonine is mainly produced industrially through microbial fermentation, typically by modifying microorganisms to increase the L-threonine yield. A variety of bacteria can be used in the production of L-threonine, such as mutant strains obtained by induction of wild-type *Escherichia*, *Corynebacterium*, *Brevibacterium*, etc., as production strains. As global demand for threonine continues to increase, the construction and modification of high-yield threonine strains are particularly important.

At present, the yield and conversion rate of amino acids are mainly increased by modifying some key genes in the amino acid metabolic pathway. Some genes at key positions can control the expression levels of enzymes and the anti-feedback ability of gene transcription. For example, CN110592084A introduces a point mutation into the *rhtA* gene of *Escherichia coli* (*E*. *coli*) K12 or its derivative strain W3110 CGMCC 7.232 to mutate the base at position 67 from adenine (A) to guanine (G), thereby improving the threonine production capacity of the recombinant strain. Jianli Wang et al. modified *E. coli* strain TWF001 by overexpressing the gene cluster *phaCAB* from *Ralstonia eutropha*, thereby increasing the L-threonine fermentation yield. Jun Yang et al. effectively increased the threonine yield by enhancing fatty acid degradation, weakening fatty acid synthesis, and accelerating the glyoxylate cycle.

However, studies on modifying genes related to the uptake of important environmental nutrients to improve the yield and conversion rate of amino acids are relatively rare. In Gram-negative bacteria, the uptake of important environmental nutrients is mainly carried out through the TonB system (TonB-dependent transporters, i.e., TBDT). TonB-dependent receptors are present in all Gram-negative bacteria. Research by Yaling Wang et al. has shown that the TonB-dependent receptor is involved not only in the uptake of transition metal elements such as iron, manganese, zinc, and nickel, as well as various nutrients such as vitamins, heme, and carbohydrates, but also in the secretion of proteases. Transition metals, as metal cofactors, play important roles in biological metabolic processes, including precursor biosynthesis, DNA replication, transcription, respiration, and oxidative stress responses. *E. coli* is a Gram-negative bacterium. In *E. coli*, genes encoding TBDT are dispersed throughout the chromosome, such as *btuB*, *fecA*, *ybiX*, and *ybiI*, and the expression of these genes is highly regulated at both the transcriptional and post-transcriptional levels. Among them, the *ybiI* gene encodes a zinc finger domain-containing protein YbiI. Such a structural domain is functionally suitable for the local structural forms on protein molecules that are involved in the interaction or matching between biological macromolecules and is one of the structural factors that ensure gene transcription and regulation in living organisms.

### SUMMARY

In the present application, whole-genome sequencing is carried out on a mutagenized strain FFTHR-1 with a high threonine yield that exists in the laboratory. It is found that a mutation occurs at position 86 in the YbiI protein, which is one of the reasons for the high threonine production of the mutagenized strain. Introducing the gene encoding the mutant protein into other production strains can also increase the threonine yield.

In a first aspect, the present application provides a mutant YbiI protein, wherein relative to a wild-type YbiI protein having a sequence as shown in SEQ ID NO: 1, the mutant YbiI protein includes at least the following mutation site: the amino acid at position 86 of the wild-type YbiI protein is mutated from glutamine to leucine.

In some embodiments, the mutant YbiI protein may further contain substitutions, deletions, insertions, additions, or inversions of other amino acids, provided that the mutation does not affect the function of the YbiI protein. In a specific embodiment, the amino acid sequence of the mutant YbiI protein is as shown in SEQ ID NO: 2.

In a second aspect, the present application provides a mutant *ybiI* gene encoding the mutant YbiI protein. The mutant *ybiI* gene may be obtained by mutation of a wild-type *ybiI* gene or may be a gene sequence designed according to the amino acid sequence.

In some embodiments, relative to a wild-type *ybiI* gene having a sequence as shown in SEQ ID NO: 3, the mutant *ybiI* gene includes at least the following mutation site: the base at position 257 of the wild-type *ybiI* gene is mutated from T to A.

In some embodiments, the mutant *ybiI* gene may also contain one or more additional base mutations, such as codon optimization mutations, conserved sequence mutations, and the like. In a specific embodiment, the nucleotide sequence of the mutant *ybiI* gene is as shown in SEQ ID NO: 4. The present application further provides the use of the mutant YbiI protein or the mutant *ybiI* gene for increasing threonine yield in a threonine-producing strain.

In a third aspect, the present application provides a high-yield L-threonine-producing recombinant strain, wherein the recombinant strain expresses the mutant YbiI protein, or the *ybiI* gene of the recombinant strain is replaced with the mutant *ybiI* gene.

In some embodiments, the *ybiI* gene of the recombinant strain is replaced with the mutant *ybiI* gene by mutagenesis, PCR site-directed mutagenesis or homologous recombination.

In some embodiments, the recombinant strain is *E*. *coli* and is preferably obtained by modification based on *E*. *coli* THRS or *E*. *coli* THRS-6. The *E*. *coli* THRS strain is the *E*. *coli* THRS strain involved in Patent CN202411834458.7 and is deposited under the deposit number CGMCC NO. 31694 on August 20, 2024 at the China General Microbiological Culture Collection Center located at NO. 3, NO. 1 West Beichen Road, Chaoyang District, Beijing, China. The *E*. *coli* THRS-6 strain is the *E*. *coli* THRS-6 strain involved in Patent CN202411834455.3 and is deposited under the deposit number CGMCC NO. 31695 on August 20, 2024 at the China General Microbiological Culture Collection Center located at NO. 3, NO. 1 West Beichen Road, Chaoyang District, Beijing, China.

In a fourth aspect, the present application further provides a method for preparing the recombinant strain described in the third aspect, wherein the method includes the following steps: designing homology arm primers PybiI-F/PybiI-R with a mutant *ybiI* gene as a template, constructing a PCR amplification product, transferring the PCR amplification product into a threonine-producing strain, and replacing a target region via homologous recombination to obtain the recombinant strain.

In a fifth aspect, the present application provides a method for producing L-threonine, wherein the method includes the following steps: inoculating a recombinant strain onto a seed medium, culturing to obtain a seed liquid, transferring the seed liquid into a fermentation medium at an inoculation quantity of 20%, and culturing to produce L-threonine.

In some embodiments, the seed medium includes 5 g/L corn steep liquor powder, 20 g/L glucose, 5 g/L yeast powder, 2 g/L KH₂PO₄, 1 g/L magnesium sulfate, 20 mg/L FeSO₄·7H₂O, and 20 mg/L MnSO₄·H₂O; the fermentation medium includes 20 g/L glucose, 2 g/L potassium dihydrogen phosphate, 3 g/L yeast powder, 1 g/L betaine, 1 g/L magnesium sulfate, 10 mg/L FeSO₄·7H₂O, 10 mg/L MnSO₄·H₂O, 8 g/L corn steep liquor powder, and 10 mg/L vitamin B1.

Compared with the existing art, the present application has at least the following beneficial effects.

The present application provides a mutant YbiI protein which can increase the threonine yield in different strains when expressed in these strains. For example, the threonine yield of the recombinant THRS strain in a 5 L fermenter is increased by 7.47%, and the threonine yield reaches 107.8 g/L after 48 hours of fermentation; the threonine yield of the recombinant THRS-6 strain in a 5 L fermenter is increased by 13.41%, and the threonine yield reaches 142.1 g/L after 48 hours of fermentation.

### DETAILED DESCRIPTION

To make the objects, technical solutions, and beneficial effects of the present application more apparent, the present application is further described in detail in conjunction with the embodiments. It should be understood that the specific examples described in the following embodiments of the present application are merely exemplary illustrations of the embodiments of the present application, are intended to explain the present application, and shall not be construed as limiting the present application.

Endpoints of the ranges and any values disclosed herein are not limited to the precise ranges or values, and such ranges or values should be understood as including values approximating to these ranges.

### Example 1: Construction of a recombinant strain using a THRS strain

The *ybiI* gene in the THRS strain was gene knockout or gene knock-in by homologous recombination. The specific steps are as follows:
Competent cells of *E*. *coli* were prepared. The pKD46 plasmid was induced with an arabinose stock solution and transferred into the competent cells of *E*. *coli*. The transferred cells were cultured and selected in an ampicillin-containing resistance medium. Homologous arms were selected at both ends of the target gene. Homology arm primers Pkan-F/Pkan-R to a kanamycin resistance gene *kan* were designed with the plasmid pKD13 as a template, and PCR amplification was carried out. The purified PCR product was transferred into a strain containing the pKD46 plasmid by electrotransformation to obtain a pKD46-kan strain. Homologous recombination occurred during the culture of the pKD46-kan strain at 30 °C to obtain a homologous recombinant strain. The pKD46 temperature-sensitive plasmid was eliminated by culturing the homologous recombinant strain at 37 °C. Resistance selection was carried out on a kanamycin-containing medium to obtain a strain with the target gene knockout. PCR validation was carried out using a primer pair PybiI-F'/PybiI-R' to confirm whether the target gene had been successfully gene knockout. Subsequently, the transferred pCP20 plasmid was introduced to express the flippase recombinase gene, thereby promoting homologous recombination at the FRT sites and finally achieving knockout of the *ybiI* gene. The *ybiI* gene knockout strain was cultured simultaneously on an LB medium and a kanamycin-containing medium. The strain that grew normally on the LB medium but did not grow on the resistance medium was identified as the *ybiI* gene knockout strain. Finally, the pCP20 temperature-sensitive plasmid was eliminated by culturing the *ybiI* gene knockout strain at 42 °C, and PCR amplification was carried out using a primer pair PpCP20-F/PpCP20-R for verification to obtain the *ybiI* gene knockout strain THRS-△*ybiI*.

Homology arm primers PybiI-F/PybiI-R were designed with the mutant *ybiI* sequence (SEQ ID NO: 4, designated as *ybiI T257A*) as a template, and a PCR amplification product of the exogenous *ybiI T257A* gene was constructed. The PCR amplification product was transferred into *E. coli* THRS and replaced a target region via homologous recombination. The specific implementation method was the same as the method described above, and finally, knock-in of the mutant *ybiI T257A* gene was achieved. The obtained recombinant strain was subjected to PCR validation and sequencing analysis. The sequencing result is shown in SEQ ID NO: 4, confirming successful obtainment of the *ybiI T257A* mutant strain THRS-YbiI Q86L.

### Example 2: L-threonine fermentation production of a recombinant strain

The *ybiI T257A* mutant strain and *ybiI* gene knockout strain obtained in Example 1 and the wild-type *THRS* strain were inoculated onto a seed medium and cultured to obtain a seed liquid, respectively. The seed medium included the following components at the indicated concentrations: 4.2 g/L corn steep liquor, 10 g/L glucose, 2.5 g/L yeast powder, 2 g/L KH₂PO₄, 1.2 g/L magnesium sulfate, 20 mg/L FeSO₄·7H₂O, 20 mg/L MnSO₄·H₂O, and 30 mg/L biotin. Three parallel replicates were set up for each set of experiments.

The seed culture obtained by the above culturing method was transferred into a fermentation medium at an inoculation quantity of 20% for culture.

### 2.1 Process control in a 5 L seed fermenter

a) The temperature was set to 37 °C, the pH was set to 7.0, the agitation speed was set to 500 rpm, and the aeration rate was set to 0.3 m³/h; the temperature was maintained at 37 °C throughout the process; the fermenter pressure was 0.05-0.08 MPa; the culture period was 10 hours.
b) Inoculation standard: OD₆₀₀: 12-15.
c) The seed medium consisted of 5 g/L corn steep liquor powder, 20 g/L glucose, 5 g/L yeast powder, 2 g/L KH₂PO₄, 1 g/L magnesium sulfate, 20 mg/L FeSO₄·7H₂O, and 20 mg/L MnSO₄·H₂O.

### 2.2 Process control for fermentation in a 5 L fermenter

a) The temperature was set to 37 °C, the pH was set to 7.0, the initial agitation speed was set to 300 rpm, and the aeration rate was set to 0.3 m³/h; the temperature was maintained at 37 °C throughout the process; the fermenter pressure was 0.05-0.08 MPa.
b) DO control: At 0 h, the aeration rate was 0.3 m³/h, the agitation speed was 300 rpm, and the fermenter pressure was 0.05 MPa.
c) When DO dropped to 30% or below, the dissolved oxygen level was controlled at 30% by adjusting the aeration rate and the stirring speed until the end of fermentation.
d) The fermentation medium consisted of 20 g/L glucose, 2 g/L potassium dihydrogen phosphate, 3 g/L yeast powder, 1 g/L betaine, 1 g/L magnesium sulfate, 10 mg/L FeSO₄·7H₂O, 10 mg/L MnSO₄·H₂O, 8 g/L corn steep liquor powder, and 10 mg/L vitamin B1.

### 2.3 Validation of production performance of the ybiI T257A strain in a 5 L fermenter

After measurement, the L-threonine fermentation yields of different strains are shown in Table 1. The results show that after glutamine at position 86 of the amino acid sequence of the *ybiI* gene is replaced with leucine, the L-threonine yield is significantly increased compared with the original strain. Meanwhile, the strain THRS-YbiI Q86L is designated as the strain FFTHR-36.

**Table 1 L-threonine production performance of strains in 5 L fermenters**

| Strain | Yield (g/L) | Conversion rate (%) |
|---|---|---|
| THRS strain | 100.3 g/L | 30.2% |
| THRS-△*ybiI* | 90.5 g/L | 31.5% |
| THRS-YbiI Q86L | 107.8 g/L | 29.7% |

### Example 3: Construction of a recombinant strain using a THRS-6 strain and L-threonine fermentation production

According to the method in Example 1, strains THRS-6-△*ybiI* and THRS-6-YbiI Q86L were constructed based on the THRS-6 strain, respectively. The yields of the original strain and the modified strains were verified by fermentation in a 5 L fermenter with reference to the fermentation method in Example 2. Meanwhile, the strain THRS-6-YbiI Q86L is designated as the strain FFTHR-37. After measurement, the L-threonine fermentation yields of different strains are shown in Table 2. The results show that the mutant YbiI protein, when used in different strains, can increase L-threonine fermentation yield.

**Table 2 L-threonine production performance of strains in 5 L fermenters**

| Strain | Yield (g/L) | Conversion rate (%) |
|---|---|---|
| THRS-6 | 125.3 g/L | 42% |
| THRS-6-△*ybiI* | 96.4 g/L | 33.2% |
| THRS-6-YbiI Q86L | 142.1g/L | 44% |

Sequences involved in the present application are as follows:
YbiI protein sequence: 
MASGWANDDAVNEQINSTIEDAIARARGEIPRGESLDECEECGAPIPQARREAIPGVRLCI
HCQQEKDLQKPAYTGYNRRGSKDSQLR (SEQ ID NO: 1).
Mutant YbiI protein sequence: 
MASGWANDDAVNEQINSTIEDAIARARGEIPRGESLDECEECGAPIPQARREAIPGVRLCI
HCQQEKDLQKPAYTGYNRRGSKDSLLR (SEQ ID NO: 2).
Nucleotide sequence of the *ybiI* gene: 
ttaacgtaactggctgtctttcgaacctctgcgattatatcctgtataagctggtttttgtaaatctttctcctgctgacaatgaatacataagcgc
acgccaggaatggcttcccgacgggcctgcgggatgggggcaccgcactcttcacattcatccaggctttcgccgcgcggaatttcaccc
cgagcgcgggcaatcgcatcttcaattgtactgttgatctgttcgttgacggcgtcatcgttagcccaaccggatgccat (SEQ ID NO:
3).
Nucleotide sequence of the mutant *ybiI* gene: 
ttaacgtaacaggctgtctttcgaacctctgcgattatatcctgtataagctggtttttgtaaatctttctcctgctgacaatgaatacataagcgc
acgccaggaatggcttcccgacgggcctgcgggatgggggcaccgcactcttcacattcatccaggctttcgccgcgcggaatttcaccc
cgagcgcgggcaatcgcatcttcaattgtactgttgatctgttcgttgacggcgtcatcgttagcccaaccggatgccat (SEQ ID NO:
4).
Pkan-F: 
5'-AAATACAAATTATACTCGCTATGCATCTCGCCAGTTGTAGAGGCTGGAGCTGCTTC-
3' (SEQ ID NO: 5).
Pkan-R: 
5'-CTACACTTAACTGTACAAGTATTGATATGGGGAGGTCGATTCCGGGGATCCGTCGA
CC-3' (SEQ ID NO: 6).
PybiI-F':
   5'-TTAACGTAACTGGCTGTCTTTCGAACCTC-3' (SEQ ID NO: 7).
PybiI-R':
   5'-ATGGCATCCGGTTGGGCTAAC-3' (SEQ ID NO: 8).
PpCP20-F:
   5'-ATGTCTGAATTAGTTGTTTTCAAAGCAAATGAAC-3' (SEQ ID NO: 9).
PpCP20-R:
   5'-GATCCTTCCGTATTTAGCCAGTATGTTCT-3' (SEQ ID NO: 10).
PybiI-F: 
5'-AAATACAAATTATACTCGCTATGCATCTCGCCAGTTGTAGTTAACGTAACAGGCTGT
CTTTCGAACCTC-3' (SEQ ID NO: 11).
PybiI-R:

5'-CTACACTTAACTGTACAAGTATTGATATGGGGAGGTCGATATGGCATCCGGTTGGG
CTAAC-3' (SEQ ID NO: 12).

Finally, it is to be noted that the above embodiments are merely used to explain the technical solutions of the present application and are not construed as limiting the content of the present application. Various simple variations can be made to the technical solutions of the present application without departing from the scope of the technical concept of the present application, including combinations of various technical features in any other suitable manners. These simple variations and combinations should be construed as content disclosed in the present application and are all within the protection scope of the present application.

## Claims

1. A mutant YbiI protein, wherein relative to a wild-type YbiI protein having a sequence as shown in SEQ ID NO: 1, the mutant YbiI protein comprises at least the following mutation site: the amino acid at position 86 of the wild-type YbiI protein is mutated from glutamine to leucine.

2. The mutant YbiI protein according to claim 1, wherein the amino acid sequence of the mutant YbiI protein is as shown in SEQ ID NO: 2.

3. A mutant *ybiI* gene encoding the mutant YbiI protein according to claim 1 or 2.

4. The mutant *ybiI* gene according to claim 3, wherein a nucleotide sequence of the mutant *ybiI* gene is as shown in SEQ ID NO: 4.

5. Use of the mutant YbiI protein according to any one of claims 1 to 2 or the mutant *ybiI* gene according to any one of claims 3 to 4 for increasing threonine yield in a threonine-producing strain.

6. A high-yield L-threonine-producing recombinant strain, wherein the recombinant strain expresses the mutant YbiI protein according to any one of claims 1 to 2, or a *ybiI* gene of the recombinant strain is replaced with the mutant *ybiI* gene according to any one of claims 3 to 4.

7. The high-yield L-threonine-producing recombinant strain according to claim 5, wherein the *ybiI* gene of the recombinant strain is replaced with the mutant *ybiI* gene by mutagenesis, PCR site-directed mutagenesis or homologous recombination.

8. The high-yield L-threonine-producing recombinant strain according to claim 7, wherein the recombinant strain is *Escherichia coli*.

9. A method for preparing the high-yield L-threonine-producing recombinant strain according to any one of claims 6 to 8, comprising: designing homology arm primers PybiI-F/PybiI-R with a mutant *ybiI* gene as a template, constructing a PCR amplification product, transferring the PCR amplification product into a threonine-producing strain, and replacing a target region via homologous recombination to obtain the recombinant strain.

10. A method for producing L-threonine, comprising: inoculating the high-yield L-threonine-producing recombinant strain according to any one of claims 6 to 8 onto a seed medium, culturing to obtain a seed liquid, transferring the seed liquid into a fermentation medium at an inoculation quantity of 20%, and culturing to produce L-threonine.
